(19) Europäisches Patentamt
European Patent Office
Office européen des brevets

(11) Numéro de publication: **0 174 887**
**B1**

(12) **FASCICULE DE BREVET EUROPÉEN**

(45) Date de publication du fascicule du brevet: **01.07.87**

(21) Numéro de dépôt: **85401609.4**

(22) Date de dépôt: **07.08.85**

(51) Int. Cl.⁴: **C 07 C 103/50**, A 61 K 31/765

(54) **Dérivés de 1- N-(alpha-amino-alpha-méthylacétyl)-aminophényl -2-amino-propanone, procédé de préparation et utilisation en thérapeutique.**

(30) Priorité: **20.08.84 FR 8412964**

(43) Date de publication de la demande: **19.03.86 Bulletin 86/12**

(45) Mention de la délivrance du brevet: **01.07.87 Bulletin 87/27**

(84) Etats contractants désignés: **AT BE CH DE FR GB IT LI LU NL SE**

(56) Documents cité:
**CH-A-348 240**
**FR-A-1 463 813**

(73) Titulaire: **LABORATOIRE L. LAFON Société anonyme dite:, 1 rue Georges Médéric, F-94701 Maisons- Alfort (FR)**

(72) Inventeur: **Lafon, Louis, 5 rue de l'Alboni, F-75016 Paris (FR)**

(74) Mandataire: **Clisci, Serge, S.A. FEDIT- LORIOT CONSEILS EN PROPRIETE INDUSTRIELLE 38, avenue Hoche, F-75008 Paris (FR)**

**EP 0 174 887 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention a trait a de nouveaux dérivés de 1-[N(α-amino-α-méthylacétyl)-aminophényl]-2-amino-propanone en tant que produits industriels, Elle concerne également le procédé de préparation et l'utilisation de ces nouveaux dérivés en thérapeutique.

On vient de trouver de façon surprenante que les nouveaux dérivés de formule I ci-après et leurs sels d'addition sont utiles en thérapeutique en tant que principes actifs agissant sur le système nerveux central, notamment eu égard à leurs effets antidépresseurs.

Les nouveaux dérivés selon l'invention sont caractérisés en ce qu'ils sont choisis parmi l'ensemble constitué par

(i) les 1-[ N-(α-alkylamino-α-méthylacétyl)-aminophényl]-2-alkylamino-propanones de formule générale

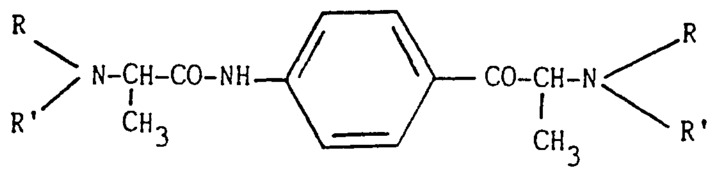

(I)

où R représente un groupe alkyle en $C_1$-$C_4$, et R' représente l'atome d'hydrogène ou un groupe alkyle en $C_1$-$C_4$; et,

(ii) leurs sels d'addition.

Parmi les groupes alkyle en $C_1$-$C_4$, qui entrent dans les définitions des groupes R et R', on peut notamment mentionner $CH_3$, $CH_2CH_3$, $CH_2CH_2CH_3$, $CH(CH_3)_2$, $CH_2CH_2CH_2CH_3$, $CH_2CH(CH_3)_2$ et $C(CH_3)_3$. De façon avantageuse selon l'invention, R représentera $CH(CH_3)_2$ et encore mieux $C(CH_3)_3$, et, R' représentera H. Les produits les plus intéressants sur le plan thérapeutique sont constitués par la 1-[4-N-(α-tertiobutylamino-α-méthylacétyl)-aminophényl]-2-tertiobutylamino-propanone et ses sels d'addition.

Par sels d'addition, on entend ici les sels d'addition d'acide obtenus par réaction d'une base libre de formule I avec un acide minéral ou organique, d'une part, et les sels d'ammonium, d'autre part. Parmi les acides utilisables pour salifier les bases libres de formule I, on peut notamment mentionner les acides chlorhydrique, bromhydrique, acétique, formique, propionique, oxalique, fumarique maléique succinique, benzoique, cinnamique, mandélique, citrique malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium.

Un certain nombre de composés selon l'invention est consigné dans le tableau I ci-après.

**Tableau 1**

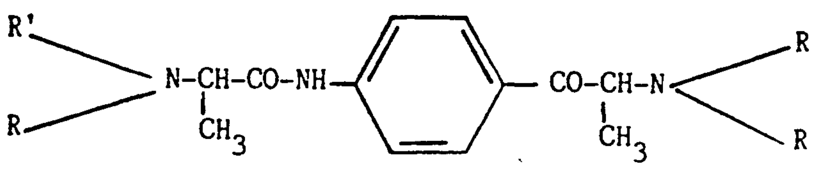

| Produit | N° de Code | R | R' |
|---|---|---|---|
| Ex 1(a) | CRL 41 275 | $C(CH_3)_3$ | H |
| Ex 2(a) | - | $CH(CH_3)_2$ | H |
| Ex 3(a) | - | $CH_3$ | $CH_3$ |
| EX 4(b) | - | $CH_2CH_3$ | H |
| Ex 5(b) | - | $C(CH_3)_3$ | H |

Notes

(a): dichlorhydrate

(b): diméthanesulfonate

Les composés de formule I peuvent être préparés selon une mméthode connue en soi par application de mécanismes réactionnels classiques. Le procédé que l'on préconise selon l'invention pour synthétiser un composé de formule I, consiste à faire réagir un dérivé dihalogéné de formule

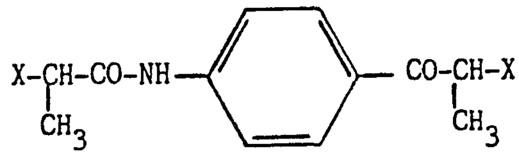

(II)

dans laquelle X représente un atome d'halogène choisi parmi F, Cl et Br, l'atome d'halogène préféré étant l'atome de chlore, avec une amine de formule

HNRR' (III)

dans laquelle R et R' sont définis comme ci-dessus.

De préférence on utilisera plus de 2 moles de III pour une mole de II, l'amine III intervenant simultanément en tant que réactif et solvant (ou co-solvant). La réaction de condensation de II avec III est mise en oeuvre pendant au moins 1 heure à une température comprise entre 15°C et la température de reflux du milieu réactionnel. De façon pratique on utilisera en tant que solvant un mélange alcool inférieur en $C_1$-$C_3$ - amine III.

Les composés de formule I selon l'invention et leurs sels sont utiles en thérapeutique notamment dans le traitement des états dépressifs, en raison de leurs propriétés stimulantes et antidépressives. Selon l'invention on préconise une composition thérapeutique qui est caractérisée en ce qu'elle renferme, en association avec un excipient physiologiquement acceptable, au moins un composé de formule I ou l'un de ses sels d'addition non-toxiques, en tant que principe actif.

Bien entendu, dans une telle composition le principe actif intervient en quantité pharmaceutiquement efficace.

D'autres avantages et caractéristiques de l'invention seront mieux compris à la lecture qui va suivre d'un exemple de préparation.

**PREPARATION**

**Obtention du dichlorhydrate de 1-[4-N-(α-tertiobutylamino-α-méthylacétyl)-aminophényl]-2-tertiobutylamino-propanone**

(Exemple 1; N° de Code: CRL 41 275)

a) α-Chloro-α-méthyl-acétanilide

On introduit goutte à goutte 18 g d'aniline dans 22,6 g de chlorure de 2-chloropropionyle, à une température de 10°C environ et sous agitation jusqu'à la fin du dégagement d'HCl. Après filtration on obtient 31g (rendement = 84,5 %) de α-chloro-α-méthylacétanilide sous forme de poudre rosée. $F_{inst.}$ = 80°C.

b) 1-[4-N(α-Chloro-α-méthyl-acétyl)-aminophényl]-2-chloro-propanone.

On introduit, à une température comprise entre 35 et 45°C, les 31 g de α-chloro-α-méthyl-acétanilide obtenus au stade a) ci-dessus dans un mélange constitué par 67 g de $AlCl_3$ et 76,8 g de chlorure de 2-chloropropionyle. Après 2 h de contact puis précipitation dans un mélange eau-glace on obtient 40 g (rendement: 74 %) de 1-[4-N-(α-chloro-α-méthylacétyl)-aminophényl ]-2-chloro-propanone.

$F_{inst.}$ = 60°C.

c) CRL 41 275

On dissout les 40 g de 1-[4-N-(α-chloro-α-méthylacétyl)-aminophényl]-2-chloro-propanone obtenus au stade b) ci-dessus dans 200 ml d'éthanol. Dans la solution résultante on ajoute 200 ml de tertiobutylamine. On chauffe au reflux pendant 8h, évapore sous vide et reprend le résidu huileux d'évaporation par 200 ml d'eau. Après extraction avec $CH_2Cl_2$ et évaporation, on dissout le résidu pâteux dans l'éthanol. Le dichlorhydrate attendu est ensuite précipité par HCl gaz. On obtient 8,5 g (rendement: 10 %) de CRL 41 275 sous forme de poudre blanc-crème. $F_{inst.}$ > 260°C.

On a résumé ci-après une partie des résultats des essais qui ont été entrepris avec le CRL 41 275 selon l'invention.

**ESSAIS RELATIFS AU CRL 41 275 (PRODUIT DE L'EXEMPLE 1)**

Dans l'étude neuropsychopharmacologique qui suit, le CRL 41 275 en solution dans de l'eau distillée (pH 5,5) a été administré par voie intrapéritonéale sous un volume de 20 ml/kg chez la souris mâle et de 5 ml/kg chez le rat mâle.

**I. TOXICITE**

Chez la souris mâle la DL-O (dose maximale non mortelle) par voie intrapéritonéale est supérieure à 128 mg/kg et la DL-30 est de l'ordre d'environ 250 mg/kg.

**II. COMPORTEMENT GLOBAL ET REACTIVITES**

Des lots de trois animaux sont observés avant, puis 0,25 h, 0,50 h, 1 h, 2 h, 3 h et 24 h après l'administration de CRL 41 275. On constate:

1. chez la souris

aux doses de 1 à 16 mg/kg:

- aucune modification nette du comportement et des réactivités;

à la dose de 64 mg/kg:

- une sédation (pour 2 animaux sur 3) après administration entre T + 1 h et T + 2 h, avec diminution de la réactivité au toucher,
- une hypothermie modérée (valeur maximale: -1,2°C) entre T + 2 h et T + 3 h; et

2. chez le rat

aux doses de 0,5 mg/kg à 32 mg/kg:

- une mydriase modérée pendant 1 à 2 h.

**III. INTERACTION AVEC L'APOMORPHINE**

1. Chez la souris

Des lots de 6 souris reçoivent le CRL 41 275 30 minutes avant l'injection sous-cutanée de 1 ou 16 mg/kg d'apomorphine. On observe que le CRL 41 275 ne modifie pas l'hypothermie induite par l'apomorphine et laisse inchangés le comportement de verticalisation et les stéréotypies.

2. Chez le rat

Le CRL 41 275 est administré à des lots de 6 rats 0,5 h avant l'injection sous-cutanée de 0,5 mg/kg d'apomorphine. On observe que le CRL 41 275 augmente faiblement l'intensité et la durée des stéréotypies induites par l'apomorphine.

**IV. INTERACTION AVEC L'AMPHETAMINE**

L'amphétamine (2 mg/kg) est injectée par voie intrapéritonéale à des lots de 6 rats. 30 minutes après l'administration du CRL 41 275. On constate que, à la dose de 32 mg/kg, le CRL 41 275 augmente fortement l'intensité et la durée des stéréotypies amphétaminiques.

**V. INTERACTION AVEC LA RESERPINE**

Quatre heures après l'injection intrapéritonéale de 2,5 mg/kg de réserpine, des lots de 6 souris reçoivent le CRL 41 275. On constate que le CRL 41 275 ne modifie pas l'hypothermie et le ptôsis réserpiniques.

**VI. INTERACTION AVEC L'OXOTREMORINE**

Le CRL 41 275 est administré à des lots de 6 souris 0,5 h avant l'injection intrapéritonéale de 0,5 mg/kg d'oxotrémorine.

1. Action sur la température

On constate que, aux deux fortes doses utilisées (16 mg/kg et 64 mg/kg), le CRL 41 275 s'oppose faiblement à l'action hypothermisante de l'oxotrémorine.

2. Action sur les tremblements

On constate que le CRL 41 275 ne modifie pas l'intensité des tremblements induits par l'oxotrémorine.

3. Action sur les symptômes cholinergiques périphériques

On observe que le CRL 41 275 ne modifie pas les signes de stimulation cholinergique périphérique induits par l'oxotrémorine.

**VII. ACTION SUR LE TEST DES QUATRE PLAQUES, LA TRACTION ET L'ELECTROCHOC**

Le test est pratiqué sur des lots de 10 souris, 30 minutes après l'administration de CRL 41 275. On constate que le CRL 41 275 ne modifie pas le nombre de passages punis, ne provoque pas d'incapacité motrice majeure, ne modifie pas les effets convulsivants mais semble aggraver les effets létaux de l'électrochoc à la dose de 64 mg/kg.

**VIII. ACTION SUR LA MOTILITE SPONTANEE**

0,5 h après avoir reçu le CRL 41 275, les souris (6 par dose, 12 témoins) sont placées en actimètre où leur motilité est enregistrée pendant 30 minutes.

On observe que, à la plus forte dose utilisée (64 mg/kg), le CRL 41 275 augmente modérément l'activité motrice spontanée de la souris.

**IX. ACTION SUR L'AGRESSIVITE INTERGROUPES**

Après avoir séjourné pendant 3 semaines dans chacune des moitiés d'une cage séparées par une cloison opaque, des groupes de 3 souris reçoivent le CRL 41 275. Une demi-heure plus tard, les deux groupes d'une même cage sont réunis par retrait de la cloison et on note le nombre de combats qui surviennent en 10 minutes. Le test est pratiqué pour moitié sur les souris habituelles (NMRI, C.E.R. Janvier) et pour moitié sur des souris NMRI (Iffa Credo).

On constate que, à la dose de 16 mg/kg, le CRL 41 275 diminue le nombre de combats.

**X. ACTION VIS-A-VIS DE QUELQUES COMPORTEMENTS PERTURBES PAR DIVERS AGENTS**

1. Motilité réduite par habituation à l'enceinte

Après 18 heures de séjour dans les actimètres, les souris (6 par dose, 12 témoins) reçoivent le CRL 41 275. Elles sont aussitôt replacées dans leurs enceintes respectives et, une demi-heure plus tard, on enregistre leur motilité pendant 30 minutes.

On observe que, aux doses de 4 et 16 mg/kg et surtout de 64 mg/kg, le CRL 41 275 entraîne une reprise nette de l'activité motrice chez la souris habituée à son enceinte.

2. Motilité réduite par agression hypoxique

Une demi-heure après avoir reçu le CRL 41 275, les souris (10 par dose, 20 témoins) sont soumises à une anoxie hypobare aiguë [dépression de 600 mmHg (i.e. environ 8 x 10 Pa) en 90 secondes; détente de 45 secondes), puis elles sont placées en actimètre où leur motilité est enregistrée pendant 10 minutes.

On observe que, aux doses de 4 mg/kg, 16 mg/kg, et surtout 64 mg/kg, le CRL 41 275 entraîne une amélioration de la récupération motrice chez la souris dont la motilité a été déprimée à la suite d'un séjour bref dans une enceinte à pression réduite. Cette amélioration est plus nette à 4 mg/kg qu'à 16 mg/kg, mais elle est surtout intense à 64 mg/kg.

3. Anoxie asphysique

Des lots de 10 souris reçoivent le CRL 41 275 une demiheure avant l'administration intrapéritonéale de 32 mg/kg de triiodoéthylate de gallamine (agent curarisant de référence).

On observe que le CRL 41 275 ne modifie pas le délai d'apparition des convulsions et de la mort consécutives à une anoxie asphyxique provoquée par un agent curarisant.

**XI. INTERACTION AVEC LE BARBITAL**

Une demi-heure après l'administration de CRL 41 275, des lots de 10 souris reçoivent une injection intrapéritonéale de barbital (220 mg/kg).

On constate que, (i) à la dose de 64 mg/kg, le CRL 41 275 augmente modérément le délai d'endormissement et diminue la durée du sommeil barbiturique, et (ii) à dose plus faible (16 mg/kg) il diminue faiblement la durée du sommeil barbiturique.

**XII. ACTION SUR LE "DESESPOIR COMPORTEMENTAL"**

Une demi-heure après avoir reçu le CRL 41 275, des lots de 6 souris sont placés dans un bécher rempli d'eau sur une hauteur de 6 cm. On note la durée totale d'immobilité entre la 2ème et la 6ème minutes suivant l'immersion.

On observe que, dès la dose de 4 mg/kg, le CRL 41 275 diminue de façon statistiquement significative la durée de l'immobilité de la souris placée en immersion forcée. La diminution de la durée de l'immobilité de désespoir est très importante à la dose de 16 mg/kg.

**XIII. CONCLUSIONS**

Il résulte de l'ensemble des essais neuropsychopharmacologiques ci-dessus que le CRL 41 275 agit dans l'organisme en tant que substance stimulante. Il présente des effets stimulants, certes modérés, mais intéressants en thérapeutique, et des effets antidépresseurs.

En clinique humaine on a obtenu de bons résultats dans le traitement des dépressions endogènes à une dose quotidienne comprise entre 20 et 50 mg per os. La posologie préconisée consiste à administrer à l'homme adulte 3 comprimés ou gélules par jour renfermant chacun 12 mg de CRL 41 275.

**Revendications**

pour les états contractants BE, CH, DE, FR, GB, IT, LI, LU, NL, SE.

1. Dérivé de 1-(N-(α-amino-α-méthylacétyl)-aminophényl]-2-amino-propanone, caractérisé en ce qu'il est choisi parmi l'ensemble constitué par

(i) les 1-(N-(α-alkylamimo-α-méthylacétyl)-amino-phényl]-2-alkylamino-propanones de formule générale

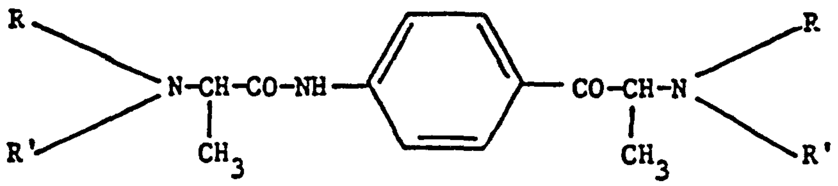

(I)

où R représente un groupe alkyle en $C_1$-$C_4$ et R' représente l'atome d'hydrogène ou un groupe alkyle en $C_1$-$C_4$; et,

(ii) leurs sels d'addition.

2. Dérivé selon la revendication 1, caractérisé en ce que R est $CH(CH_3)_2$ ou $C(CH_3)_3$.

3. Dérivé selon la revendication 1, caractérisé en ce que R' est H.

4. 1-[4-N-(α-Tertiobutylamino-α-méthylacétyl)-amino-phényl]-2-tertiobutylamino-propanone et ses sels d'addition.

5. Composition thérapeutique, caractérisée en ce qu'elle renferme, en association avec un excipient physiologiquement acceptable, au moins un dérivé de formule I selon la revendication 1 ou l'un de ses sels d'addition non-toxiques.

6. Composition thérapeutique selon la revendication 5, caractérisée en ce qu'elle renferme la 1-[4-N-(α-tertiobutylamino-α-méthylacétyl)-aminophényl)-2-tertiobutylamino-propanone ou l'un de ses sels d'addition non-toxiques.

7. Procédé de préparation d'un dérivé de formule I selon la revendication 1, caractérisé en ce que l'on condense un dérivé dihalogéné de formule

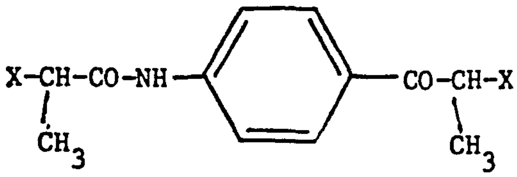

(II)

dans laquelle X est F, Cl ou Br, avec une amine de formule
HNRR' (III)
dans laquelle R et R' sont définis comme indiqué ci-dessus.

8. Procédé selon la revendication 7, caractérisé en ce que l'on fait réagir 1 mole de dérivé dihalogéné II avec plus de 2 moles d'amine III, à une température comprise entre 15°C et la température de reflux du milieu réactionnel.

9. Utilisation d'un dérivé de 1-[N-(α-amino-α-méthyl-acétyl)-aminophényl]-2-amino-propanone de formule 1 selon la revendication 1, pour l'obtention d'un médicament stimulant et antidépresseur du SNC destiné à un usage thérapeutique vis-à-vis des dépressions endogènes.

**Revendications**

pour l'état contractant: AT.

1. Procédé de préparation d'un dérivé de 1-[N-(α-amino-α-méthylacétyl)-amimophényl]-2-amino-propanone choisi parmi l'ensemble comprenant
(i) les 1-[N-(α-alkylamino-α-méthylacétyl)-amino-phényl]-2-alkylamino-propanones de formule générale

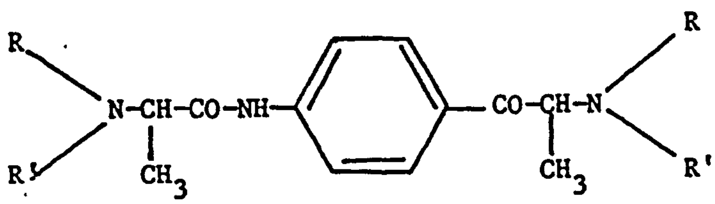

(I)

où R représente un groupe alkyle en $C_1$-$C_4$ et R' représente l'atome d'hydrogène ou un groupe alkyle en $C_1$-$C_4$; et
(ii) leurs sels d'addition,
ledit procédé étant caractérisé en ce que l'on condense un dérivé dihalogéné de formule

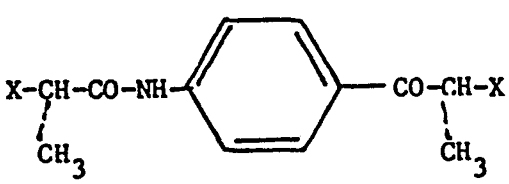

(II)

dans laquelle X est F, Cl ou Br, avec une amine de formule
HNRR' (III)
où R et R' sont définis comme indique ci-dessus,

2. Procédé selon la revendication 1, caractérise en ce que l'on fait réagir 1 mole de dérivé dihalogéné II avec plus de 2 moles d'amine III, à une température comprise entre 15°C et la température de reflux du milieu réactionnel.

3. Procédé selon l'une quelconque des revendications 1 et 2, caractérisé en ce que R est $CH(CH_3)_2$ ou $C(CH_3)_3$.

4. Procédé selon l'une quelconque des revendications 1 et 2, caractérisé en ce que R est $C(CH_3)_3$ et R' est H.

**Patentansprüche**

für die Vertragsstaaten: BE, CH, DE, FR, GB, IT, LI, LU, NL, SE

1. Derivat des 1-[N-(α-Anino-α-methylacetyl)-aminophenyl]-2-aminopropanon, dadurch gekennzeichnet, daß

es aus der Gruppe ausgewählt ist, bestehend aus

(i) 1-[N-(α-Alkylamino-α-methylacetyl)aminophenyl]-2-alkylaminopropanonen der allgemeinen Formel

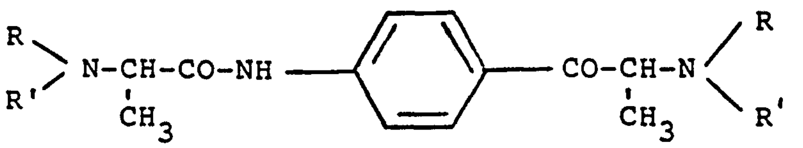

(I)

worin

R eine $C_1$-$C_4$-Alkylgruppe und

R' ein Wasserstoffatom oder eine $C_1$-$C_4$-Alkyl-gruppe bedeutet, und

(ii) deren Additionssalze.

2. Derivat nach Anspruch 1, dadurch gekennzeichnet, daß R für $CH(CH_3)_2$ oder $C(CH_3)_3$ steht.

3. Derivat nach Anspruch 1, dadurch gekennzeichnet, daß R' für H steht.

4. 1-[4-N-(α-tert.-Butylamino-α-methylacetyl)amino-phenyl]-2-tert.-butylaminopropanon und deren Additionssalze.

5. Therapeutikum, dadurch gekennzeichnet, daß es zusammen mit einem geeigneten physiologischen Excipienten wenigstens ein Derivat der Formel (I) nach Anspruch 1 oder eines ihrer nicht-toxischen Additionssalze enthält.

6. Therapeutikum nach Anspruch 5, dadurch gekennzeichnet, daß es 1-[4-N-(α-tert.-Butylamino-α-methylacetyl)-aninophenyl]-2-tert.-butylaminopropanon oder eines seiner nicht-toxischen Additionssalze enthält.

7. Verfahren zur Herstellung eines Derivats der Formel (I) nach Anspruch 1, dadurch gekennzeichnet, daß ein Dihalogenderivat der Formel

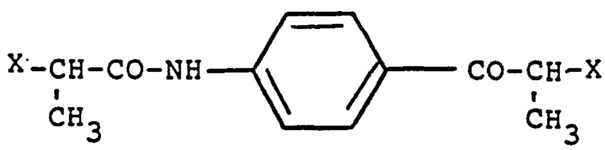

(II)

in der X für F, Cl oder Br steht, mit einem Amin der Formel

HNRR' (III)

kondensiert wird, in der R und R' im vorstehender Weise definiert sind.

8. Verfahren nach Anspruch 7, dadurch gekennzeichmet, daß 1 Mol des Dihalogenderivates (II) mit mehr als 2 Mol des Amins (III) bei einer Temperatur zwischen 15°C einschließlich und der Rückflußtemperatur des Reaktionsmediums zur Reaktion gebracht wird.

9. Verwendung eines Derivates von 1-[N-(α-Amino-α-methyl-acetyl)-aminophenyl]-2-aminopropanon der Formel (I) nach Anspruch 1 für ein stimulierendes und antidepressives Medikament für das ZNS zur Therapie von endogenen Depressionen.

**Patentansprüche**

für den Vertragsstaat AT

1. Verfahren zur Herstellung eines Derivates von 1-[N-(α-Amino-α-methylacetyl)-aminophenyl]-2-aminopropanon ausgewählt aus der Gruppe, bestehend aus

(i) 1-[N-(α-Alkylamino-α-methylacetyl)-aminophenyl]-2-alkylaminopropanonen der allgemeinen Formel

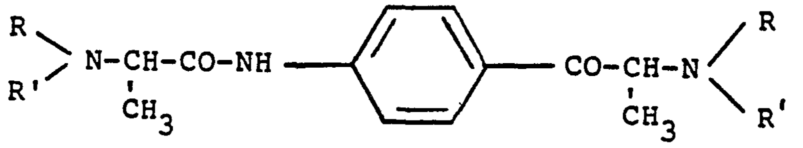

(I)

in der R eine $C_1$-$C_4$-Alkylgruppe und

R' ein Wasserstoffatom oder eine $C_1$-$C_4$-Alkylgruppe bedeutet, und

(ii) ihren Additionssalzen,

wobei das Verfahren dadurch gekennzeichnet ist, daß ein Dihalogenderivat der Formel

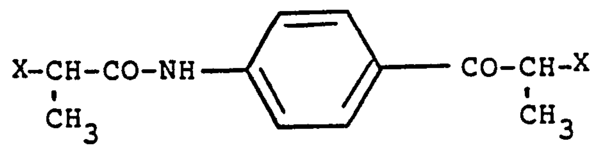

(II)

in welcher X für F, Cl oder Br steht, mit einem Amin der Formel
HNRR' (III)
kondensiert, in der R und R' wie vorstehend definiert sind.

2. Verfahren nach Anspruch 1, dadurch gekennzeichnet, daß 1 Mol des Dihalogenderivates (II) mit mehr als 2 Mol des Amins (III) bei einer Temperatur zwischen 15°C einschließlich und der Rückflußtemperatur des Reaktionsmediums zur Reaktion gebracht wird.

3. Verfahren nach einem der Ansprüche 1 und 2, dadurch gekennzeichnet, daß R für $CH(CH_3)_2$ oder $C(CH_3)_3$ steht.

4. Verfahren nach einem der Ansprüche 1 und 2, dadurch gekennzeichnet, daß R für $C(CH_3)_3$ und R' für H steht.

**Claims**

for the contracting states: BE, CH, DE, FR, GB, IT, LI, LU, NL, SE.

1. A 1-[N-(α-amino-α methylacetyl)aminophenyl]-2-aminopropanone derivative selected from the group consisting of:
(i) 1-[N-(α-alkylamino-α-methylacetyl)amino-phenyl)-2-alkylaminopropanones of the general formula:

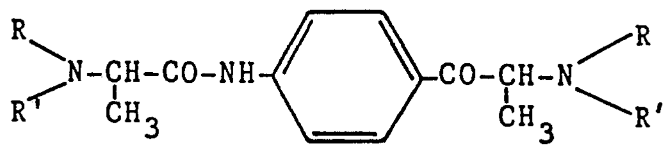

(I)

in which R represents $C_1$-$C_4$ alkyl and R' represents hydrogen or $C_1$-$C_4$-alkyl; and
(ii) addition salts thereof.

2. A derivative according to claim 1, wherein R is $CH(CH_3)_2$ or $C(CH_3)_3$.

3. A derivative according to claim 1, wherein R' is H.

4. 1-[4-N-(α-Tert.-butylamino-α-methylacetyl)amino-phenyl]-2-tert.-butylaminopropanone and its non-toxic addition salts.

5. A therapeutic composition comprising, in association with a physiologically acceptable excipient, a pharmaceutically effective quantity of a derivative of the formula 1 according to claim 1, or a non-toxic addition salt thereof.

6. A therapeutic composition according to claim 5, comprising a pharmaceutically effective quantity of 1-[4-N-(α-tert.-butylamino-α-methylacetyl)aminophenyl]-2-tert.-butylaminopropanone or one of its non-toxic addition salts.

7. A method for the preparation of a derivative of the formula 1 according to claim 1, which comprises reacting a dihalogen derivative of the formula:

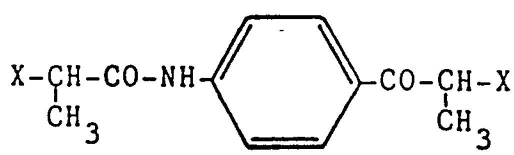

(II)

in which X represents F, Cl or Br, with an amine of the formula:
HNRR' (III)
in which R and R' are as defined above.

8. The method according to claim 7, wherein 1 mol of II is reacted with more than 2 mol of III at a temperature of between 15° C and the reflux temperature of the reaction medium.

9. The use of a 1-[N-(α-alkylamino-α-methylacetyl)aminophenyl]-2-alkylaminopropanone derivative of the formula I according to claim 1, for obtaining a CNS-stimulant and antidepressant medicament to be used in therapy against endogenic depressions.

**Claims**

for the contracting State AT

1. A method for the preparation of a 1-[N-(α-amino-α-methylacetyl)aminophenyl]-2-aminopropanone derivative selected from the group consisting of:
(i) 1-[N-(α-alkylamino-α-methylacetyl)amino-phenyl]-2-alkylaminopropanones of the general formula:

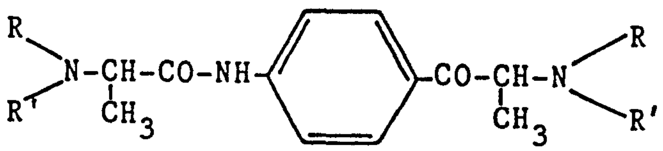
(I)

in which R represents $C_1$-$C_4$ alkyl and R′ represents hydrogen or $C_1$-$C_4$-alkyl; and
(ii) addition salts thereof, the said method comprising reacting a dihalogen derivative of the formula:

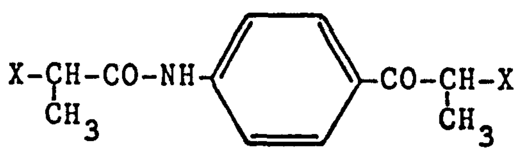
(II)

in which X represents F, Cl or Br, with an amine of the formula:
HNRR′ (III)
in which R and R′ are as defined above.
2. The method according to claim 1, wherein 1 mol of II is reacted with more than 2 mol of III at a temperature of between 15° C and the reflux temperature of the reaction medium.
3. The method according to claim 1 or 2, wherein R is $CH(CH_3)_2$ or $C(CH_3)_3$.
4. The method according to claim 1 or 2, wherein R is $C(CH_3)_3$ and R′ is H.